Europäisches Patentamt

European Patent Office

Office européen des brevets

(19)

(11) Veröffentlichungsnummer: **0 247 479**
**B1**

(12) **EUROPÄISCHE PATENTSCHRIFT**

(45) Veröffentlichungstag der Patentschrift: **08.08.90**

(51) Int. Cl.⁵: **C 07 D 209/88**

(21) Anmeldenummer: **87107158.5**

(22) Anmeldetag: **18.05.87**

(54) **Verfahren zur Herstellung von 2-Hydroxy-carbazol-1-carbon-säurenatriumsalz.**

(30) Priorität: **28.05.86 DE 3618034**

(43) Veröffentlichungstag der Anmeldung:
**02.12.87 Patentblatt 87/49**

(45) Bekanntmachung des Hinweises auf die
Patenterteilung:
**08.08.90 Patentblatt 90/32**

(84) Benannte Vertragsstaaten:
**CH DE FR GB IT LI NL**

(56) Entgegenhaltungen:
**EP-A-0 110 239**
**EP-A-0 181 546**
**DE-C- 512 234**

(73) Patentinhaber: **BAYER AG**
**D-5090 Leverkusen 1 Bayerwerk (DE)**

(72) Erfinder: **Blank, Heinz-Ulrich, Dr.**
**Am Geusfelde 35**
**D-5068 Odenthal (DE)**
Erfinder: **Meier, Jürgen-Dietrich, Dr.**
**Tempelhoferstrasse 22**
**D-5090 Leverkusen (DE)**
Erfinder: **Bauer, Wolfgang, Dr.**
**Walter-Flex-Strasse 32**
**D-5090 Leverkusen (DE)**

EP 0 247 479 B1

Courier Press, Leamington Spa, England.

**Beschreibung**

Aus FIAT Final Report Nr. 1313, Seite 364 ist die Umsetzung von 2-Hydroxy-carbazolkaliumsalze mit $CO_2$ bei 180—260°C und 4,5 bar Druck zu einem Isomerengemisch von 2-Hydroxy-carbazol-1-carbonsäure (2,1-Azylsäure) und 2-Hydroxy-carbazol-3-carbonsäure (2,3-Azylsäure) bekannt. Die Isolierung der gewüngschten 2,1-Azylsäure erfolgt durch Eintragen des aus Carboxylierung erhaltenen Reaktionsgemisches in Wasser, Neutralisation mit $H_2SO_4$, Abfiltration von nicht umgesetztem 2-Hydroxycarbazol und Aussalzen der 2,1-Azylsäure in Form ihres Natriumsalzes mit Hilfe von NaCl. Der Nachteil dieses Verfahrens besteht im großen Anteil an isomerer 2,3-Azylsäure, die gemeinsam mit einem Teil der 2,1-Azylsäure in das Filtrat gelangt.

In EP 110 239 wird ein verbessertes Verfahren zur selektiveren Herstellung der 2,1-Azylsäure beschrieben, das in Gegenwart eines Alkanols durchgeführt wird. Hierbei wird ebenfalls das wegen seiner höheren Löslichkeit vielfach unerwüschte Kaliumsalz erhalten; dieses Kaliumsalz fällt fernerhin in Alkanolfeuchter Form an und muß daher zunächst in unwritschaftlicher Form getrocknet und sodann als trockenes Kaliumsalz gehandhabt werden.

Es wurde nun ein Verfahren zur Herstellung von 2-Hydroxycarbazol-1-carbonsäurenatriumsalz (2,1-Azylsäurenatriumsalz) durch Umsetzung von 2-Hydroxy-carbazol-1-carbonsäurekaliumsalz mit NaCl in wäßrigem Medium gefunden, das dadurch gekennzeichnet ist, daß man die Umsetzung in Gegenwart von 1,5—6,0 Gew.-%, bezogen auf die Wassermenge, eines $C_3$—$C_{10}$-Alkanols und bei einem pH-Wert zwischen 8 und 14 durchführt und 4 bis 10 Mol NaCl pro Mol 2-Hydroxycarbazol-1-carbonsäure kaliumsalz einsetzt.

Alkanole für das erfindungsgemäße Verfahren sind aliphatische geradkettige oder verzweigte Monohydroxyverbindungen mit 3 bis 10, bevorzugt 3 bis 8, besonders bevorzugt 3 bis 6 C-Atomen, wie Propanol, Isopropanol, Butanol, Isobutanol, tert.-Butanol, Pentanol, Hexanole, Octanole oder Decanole, die selbstverständlich auch als Gemische untereinander eingesetzt werden können. In ganz besonders bevorzugter Weise wird mit den genannten Propanolen und Butanolen gearbeitet.

Die genannten Alkanole werden in einer Menge von 1,5 bis 6,0 Gew.-%, bevorzugt 1,7 bis 5,0 Gew.-%, besonders bevorzugt 1,9 bis 4,3 Gew.-%, bezogen auf die Wassermenge des gesamten Umsetzungsgemisches, eingesetzt. Das Arbeiten außerhalb der genannten Mengenbereiche ist grundsätzlich möglich, kann aber zu etwas schlechteren Ergebnissen führen.

Das erfindungsgemäße Verfahren wird bei einem pH-Wert von größer als 8 durchgeführt, wie bei einem pH-Wert zwischen 8 und 14, bevorzugt bei 8,3 bis 12, besonders bevorzugt bei 8,5 bis 11,5 und ganz besonders bevorzugt bei 8,7 bis 10,5.

Das zur erfindungsbemäßen Umsetzung erfoderliche NaCl kann beispielweise in chemisch reiner oder technischer Qualität, etwa als gewöhnliches Steinsalz, in einer Menge von 4 bis 10 Mol, bevorzugt 4 bis 6 Mol pro Mol des Azylsäurekaliumsalzes eingesetzt werden.

Das NaCl kann hierzu in fester oder gelöster Form, beispielsweise als gesättigte wäßrige Lösung zugesetzt werden. Vor, während oder nach dem Zusatz von NaCl kann das gesamte Umsetzungsgemisch eine Temperatur von Raumtemperatur bis zur Siedetemperatur des Reaktionsgemisches haben oder auf eine solche Temperatur gebracht werden. Nach dem Zusatz des NaCl läßt man das Umsetzungsgemisch auf eine Temperatur von höchstens 60°C, beispielsweise 0 bis 60°C, bevorzugt 5 bis 50°C, besonders bevorzugt 10 bis 40°C abkühlen und gewinnt das 2,1-Azylsäurenatriumsalz durch Filtration. Falls erfoderlich, kann in üblicher Weise vor Beginn der Kristallisation eine Klärfiltration vorgenommen werden.

Das erfindungsgemäße Verfahren kann beispielweise so durchgeführt werden, daß man das rohe, aus der Carboxylierungsreaktion stammende Azylsäurekaliumsalz (beispielsweise das oben beschriebene Isomerengemisch) mit Wasser und der erfindungsgemäßen Menge eines oder mehrerer der obengenannten Alkanole versetzt, dieses Gemisch zur Auflösung des rohen Azylsäurekaliumsalzes bringt, anschließend mit dem NaCl versetzt und dann zur Kristallisation bringt; gegebenenfalls werden vor und/oder nach der NaCl-Zugabe Klärfiltrationen durchgeführt.

Für den Fall, daß die Carboxylierung des 2-Hydroxycarbazolkaliumsalzes mit $CO_2$ gemäß der oben erwähnten EP 110 239 in einem alkanolischen Medium durchgeführt wird, kann des erfindungsgemäße Verfahren bevorzugt so ausgeführt werden, daß man dieses gemäß EP 110 239 erhaltene alkanolische Carboxyleirungsgemisch zunächst mit Wasser versetzt, sodann das Alkanol bis auf einen Restgehalt von 1 bis 4,5 Gew.-%, bezogen auf das durch die Destillation erhaltene Gesamtgemisch, abdestilliert und dann weiter in der oben beschriebenen Weise verfährt.

Falls das Umsetzungsgemisch nicht den genannten pH-Wert aufweist, kann dieser durch geeignet reagierende Stoffe, wie NaOH, KOH, $Na_2CO_3$, $K_2CO_3$, HCl, $H_2SO_4$ oder Essigsäure eingestellt werden.

Das 2-Hydroxy-carbazol-1-carbonsäurenatriumsalz wird erfindungsgemäß in gut kristallisierter Form sowie in hoher Ausbeute und hoher Reinheit erhalten. Durch die gute Kristallisation enthält der bei der Isolierung des Kristallisats erhaltene Filterkuchen vergleichsweise erhöhte Feststoffgehalte (Trockenmasse). Die Austauschrate von Natrium gegen Kalium erreicht deutlich höhere Werte als bei bekannten Verfahen; diese Werte liegen in der Regel über 97%, vielfach über 99%.

Aus dem Natriumsalz kann durch Zusatz von Mineralsäure die freie 2-Hydroxy-carbazol-1-carbonsäure gewonnen werden. Das Natriumsalz kann jedoch auch als solches weiterverarbeitet werden, beispielsweise zur Herstellung von Naphthol-AS-Farbstoffen.

Die folgenden Beispiele erläutern die Erfindung.

### Beispiele 1 bis 5

Alle Reaktionsansätze (jeweils 0,34 Mol) wurden in Planschliffbechern, versehen mit einem Balkenrührer, einem Thermofühler und einem Rückflußkühler bzw. einem Destillationsaufsatz, durchgeführt. Nach dem Abkühlen wurde das Reaktionsgut über eine Porzellannutsche (Durchmesser 12,5 cm) bei einem Vakuum von ca. 30 kPa in ca. 3 min abgesaugt. Danach wurde 2 mal mit je 75 ml 10 Gew.-%iger NaCl-Lösung gewaschen und bei ca. 3 cm Schichtdicke des Nutschkuchens 15 min trockengesaugt. Die weitere Trocknung erfolgte im Trockenschrank.

### Beispiel 1

0,34 Mol 2-Hydroxy-carbazol-1-carbonsäure-kaliumsalz (2,1-Azylsäurekaliumsalz, 96%ige Ware), 15,2 g Butanol und 740 ml Wasser wurden auf ca. 95°C erhitzt und mit 99 g NaCl (= 1,7 Mol) versetzt.

Der pH-Wert der Lösung lag bei 8,9. Nach dem Abkühlen unter Rühren auf Raumtemperatur wurde ein grobes, ausgeprägtes Kristallisat erhalten, das sich beim Abstellen des Rührers spontan absetzte. Die charackteristischen Versuchsdaten befinden sich in Tabelle 1.

### Beispiel 2

832 ml 2,1-Azylsäurekaliumsalz-Suspension (0,34 Mol 2,1-Azylsäurekaliumsalz 100%ig, 787 ml Wasser, ca. 2,4% Restbutanol, bezogen auf die Wassermenge) wurden auf ca. 100°C erhitzt und mit 105 g NaCl (= 1,8 Mol) versetzt; der pH-Wert der Lösung lag bei etwa 10. Nach dem Abkühlen auf Raumtemperatur wurde ein grobes, ausgeprägtes Kristallisat erhalten, das sich beim Abstallen des Rührers spontan absetzte. Die charakteristischen Versuchsdaten stehen in Tabelle 1.

### Beispiel 3 (Vergleichsbeispiel)

0,34 Mol 2,1-Azylsäurekaliumsalz und 740 ml Wasser wurden auf 80°C erhitzt, 90 g NaCl (= 1,5 Mol) zugesetzt, der pH-Wert lag bei 9. Nach dem Abkühlen auf Raumtemperatur wurde eine dickflüssige Suspension erhalten (Daten siehe Tabelle 1).

### Beispiel 4 (Vergleichsbeispiel)

0,34 Mol 2,1-Azylsäurekaliumsalz (96%ige Ware), 72,5 g Butanol und 740 ml Wasser wurden auf ca. 95°C erhitzt und mit 99 g NaCl (= 1,7 Mol) versetzt; der pH-Wert der Lösung lag bei ca. 9. Nach dem Abkühlen auf Raumtemperatur wurde erst nach längerem Stehen ein schlecht verarbeitbares Festmaterial erhalten (Daten siehe Tabelle 1).

### Beispiel 5 (Vergleichsbeispiel)

838 ml 2,1-Azylsäurekaliumsalzsuspension (0,34 Mol 2,1-Azylsäurekaliumsalz 100%ig, 789 ml Wasser ca. 2,4% Restbutanol, bezogen auf die Wassermenge) wurden auf ca. 95°C erhitzt und mit 105 g NaCl (= 1,8 Mol) versetzt; der pH der Lösung wurde mit verdünnter Schwefelsäure auf 8,0 eingestellt. Nach Abkühlen auf Raumtemperatur beobachtete man eine Suspension mit wenig ausgeprägten Kristallen (Daten siehe Tabelle 1).

### TABELLE 1
### (Beispiele 1—5)

| Bsp. Nr. | pH-Wert | BuOH-Gehalt (%, bezogen auf die $H_2O$-Menge) | Sedimentation | Gew.-% Trocken-material im Filterkuchen | Na-Gehalt (Mol-%, bezogen auf Na + K) |
|---|---|---|---|---|---|
| 1 | 8,9 | 2,1 | gut | 76 | 99 |
| 2 | ~10 | 2,4 | gut | 79 | >99 |
| 3 (Vergleich) | 9 | 0 | schlecht | 60 | 91 |
| 4 (Vergleich) | ~9 | 9,8 | schlecht | 51 | 88 |
| 5 (Vergleich) | 8,0 | 2,4 | schlecht | 66 | 93 |

Beispiele 6—12

Die Beispiele 6—12 wurden analog Beispiel 2 durchgeführt mit dem Unterschied, daß in Tabelle 2 aufgeführten Alkanole in einer Menge von 3,2 Gew.-%, bezogen auf die Wassermenge, eingesetzt wurden.

Beispiel 13 und 14 (Vergleichsbeispiele)

Es wurde wie bei den Beispielen 6—12 verfahren, nur daß bei Beispiel 13 Methanol und bei Beispiel 14 Ethanol zugesetzt wurde.

TABELLE 2 (Beispiele 6—14)

| Beispiel Nr. | Alkanol | Sedimentation | Gew.-% Trockenmaterial im Filterkuchen |
|---|---|---|---|
| 6 | n-Propanol | gut | 80 |
| 7 | i-Propanol | gut | 76 |
| 8 | i-Butanol | gut | 77 |
| 9 | t-Butanol | gut | 75 |
| 10 | n-Pentanol | gut | 83 |
| 11 | n-Octanol | gut | 75 |
| 12 | n-Decanol | gut | 76 |
| 13 (Vergleich) | Methanol | schlecht | 43 |
| 14 (Vergleich) | Ethanol | schlecht | 43 |

## Patentansprüche

1. Verfahren zur Herstellung von 2-Hydroxy-carbazol-1-carbonsäurenatriumsalz (2,1-Azylsäurenatriumsalz) durch Umsetzung von 2-Hydroxy-carbazol-1-carbonsäurekaliumsalz mit NaCl in wäßrigem Medium, dadurch gekennzeichnet, daß man die Umsetzung in Gegenwart von 1,5—6,0 Gew.-%, bezogen auf die Wassermenge, eines $C_3$—$C_{10}$-Alkanols und bei einem pH-Wert zwischen 8 und 14 durchführt und 4 bis 10 Mol NaCl pro Mol 2-Hydroxy-carbazol-1-carbonsäure kaliumsalz einsetzt.

2. Verfahren nach Anspruch 1, dadurch gekennzeichnet, daß in Gegenwart eines $C_3$—$C_8$-Alkanols gearbeitet wird.

3. Verfahren nach Ansprüchen 1 und 2, dadurch gekennzeichnet, daß in Gegenwart eines $C_3$—$C_6$-Alkanols gearbeitet wird.

4. Verfahren nach Ansprüchen 1 bis 3, dadurch gekennzeichnet, daß der Alkanolgehalt bei 1,7 bis 5,0 Gew.-%, bezogen auf die Wassermenge, liegt.

5. Verfahren nach Ansprüchen 1—4, dadurch gekennzeichnet, daß der Alkanolgehalt bei 1,9 bis 4,3 Gew.-%, liegt.

6. Verfahren nach Ansprüchen 1—5, dadurch gekennzeichnet, daß der pH-Wert bei 8,3 bis 12, liegt.

7. Verfahren nach Ansprüchen 1—6, dadurch gekennzeichnet, daß der pH-Wert bei 8,5 bis 11,5 liegt.

8. Verfahren nach Ansprüchen 1—7, dadurch gekennzeichnet, daß der pH-Wert bei 8,7 bis 10,5 liegt.

9. Verfahren zur Herstellung von 2-Hydroxy-carbazol-1-carbonsäurenatriumsalz (2,1-Azylsäurenatriumsalz) nach Anspruch 1, dadurch gekennzeichnet, daß man ein alkanolisches Carboxylierungsgemisch mit Wasser versetzt, das Alkanol bis zu einem Restgehalt von 1,5 bis 6,0 Gew.-%, bezogen auf die Wassermenge, abdestilliert und dann weiter nach Anspruch 1 verfährt.

## Revendications

1. Procédé pour la fabrication de 2-hydroxycarbazole-1-carboxylate de sodium (2,1-azylate de sodium) par réaction du 2-hydroxycarbazole-1-carboxylate de potassium avec NaCl en milieu aqueux, caractérisé en ce que l'on met en oeuvre la réaction en présence de 1,5—6,0% en poids d'un alcanol en $C_3$—$C_{10}$, par rapport à la quantité d'eau, et à un pH compris entre 8 et 14 et on utilise 4 à 10 mol de NaCl par mol de 2-hydroxycarbozole-1-carboxylate de potassium.

2. Procédé selon la revendication 1, caractérisé en ce que l'on opère en présence d'un alcanol en $C_3—C_8$.

3. Procédé selon les revendications 1 et 2, caractérisé en ce que l'on opère en présence d'un alcanol en $C_3—C_6$.

4. Procédé selon les revendications 1 et 3, caractérisé en ce que la teneur en alcanol est de 1,7 à 5,0% en poids par rapport à la quantité d'eau

5. Procédé selon les revendications 1—4, caractérisé en ce que la teneur en alcanol est de 1,9 à 4,3% en poids.

6. Procédé selon les revendications 1—5, caractérisé en ce que le pH est de 8,3 à 12.

7. Procédé selon les revendications 1—6, caractérisé en ce que le pH est de 8,5 à 11,5.

8. Procédé selon les revendications 1—7, caractérisé en ce que le pH est de 8,7 à 10,5.

9. Procédé pour la fabrication de 2-hydroxycarbazole-1-carboxylate de sodium (2,1-azylate de sodium) selon la revendication 1, caractérisé en ce que l'on ajoute de l'eau à un mélange alcanolique de carboxylation, on chasse l'alcanol par distillation jusqu'à teneur résiduelle de 1,5 à 6,0% en poids par rapport à la quantité d'eau et on procède ensuite selon la revendication 1.

**Claims**

1. Process for the preparation of sodium 2-hydroxycarbazole-1-carboxylate (sodium 2,1-azylate) by reaction of potassium 2-hydroxy-carbazole-1-carboxylate with NaCl in aqueous medium, characterized in that the reaction is carried out in the presence of 1.5—6.0% by weight, relative to the amount of water, of a $C_3—C_{10}$-alkanol and at a pH between 8 and 14, and 4 to 10 moles of NaCl are employed per mole of potassium 2-hydroxy-carbazole-1-carboxylate.

2. Process according to Claim 1, characterized in that it is carried out in the presence of a $C_3—C_8$-alkanol.

3. Process according to Claims 1 and 2, characterized in that it is carried out in the presence of a $C_3—C_6$-alkanol.

4. Process according to Claims 1 to 3, characterized in that the alkanol content is 1.7 to 5.0% by weight, relative to the amount of water.

5. Process according to Claims 1—4, characterized in that the alkanol content is 1.9 to 4.3% by weight.

6. Process according to Claims 1—5, characterized in that the pH is 8.3 to 12.

7. Process according to Claims 1—6, characterized in that the pH is 8.5 to 11.5.

8. Process according to Claims 1—7, characterized in that the pH is 8.7 to 10.5.

9. Process for the preparation of sodium 2-hydroxycarbazole-1-carboxylate (sodium 2, 1-azylate) according to Claim 1, characterized in that water is added to an alkanolic carboxylation mixture, the alkanol is removed by distillation to a residual content of 1.5 to 6.0% by weight, relative to the amount of water, and a procedure according to Claim 1 is then followed.